# EUROPEAN PATENT APPLICATION

(11) **EP 2 233 895 A1**
(43) Date of publication of application: **29.09.2010**
(21) Application number: 07873381.3
(22) Date of filing: 25.12.2007
(51) Int. Cl.: G01F 1/42, G01N 1/10, G01N 1/22

(54) **METHOD AND DEVICE FOR FLOW METERING AND FOR FORMING A FLUID MEDIUM SAMPLE**

(71) Applicant: OG Systems Limited, Victoria, Mahe (SC)
(72) Inventor: STARIKOV, Vladislav Petrovich, Moskovskaya obl. 143430 (RU)
(74) Representative: Thoma, Michael
(86) International application number: PCT/RU2007/000731
(87) International publication number: WO 2009/082253

(57) **Abstract**

The invention relates to measuring quantity and composition of gas and liquids transported via pipelines. The inventive method for flow metering and forming a fluid medium sample in a pipeline involves passing a fluid medium flow through the grid of a straightening vane, a nozzle and a diffuser, wherein, on a path between the straightening vane and the nozzle, the portion of fluid medium enters the first static high pressure measuring cavity, on the section downstream of the nozzle, the portion of fluid medium enters the second static low-pressure measuring pressure cavity, and, prior to the diffuser, the portion of fluid medium enters a side fluid medium sample forming cavity for subsequently determining the composition of said sample. The inventive device for flow metering and forming a fluid medium sample comprises the following components in series arranged along a common axis: the straightening vane, a cylindrical high-pressure chamber, the nozzle, a low-pressure chamber, a sampling chamber and the diffuser. The high-pressure chamber is coupled to an annular high-pressure measuring chamber which embraces it, the low-pressure chamber is coupled to an annular low-pressure measuring chamber which embraces it, and the sampling chamber is coupled to an annular chamber which is used for additionally stirring samples and embraces said sampling chamber.

## Description

The invention relates to measuring the quantity and composition of gas and liquids transported via pipelines by formation of differential pressure when passing the fluids through constriction means. The invention can be used as part of control equipment on oil and gas pipelines, including trunk pipelines.

A conventional gas flow meter includes arranged successively on a common axis: a cylindrical section having a straightening vane, a nozzle, a cylindrical branch pipe and a conical fitting, as well as annular chambers equipped with high and low pressure sensors that are arranged coaxially with the section encompassed by the cylindrical section and the cylindrical branch pipe and communicate, through longitudinal slots, with internal cavities thereof (WO 98/48246, publ.29.10.1998).

Another gas flow meter of a similar design comprises a low pressure measuring section flaring conically towards the inlet of a conical fitting (RU 2186341, publ.27.02.2002).

However, in both devices the fact is neglected that the metering accuracy is influenced by the deformation component appearing due to the pressure difference between the cylindrical section and the inner pressure in the flow at the nozzle outlet and on the way to the low-pressure measuring section in the enclosing cavity of the cylindrical branch pipe, as well as the potential for a wall layer and flow separation due to resonant oscillations of the wall layer caused by turbulent phenomena in the flow in this section. This fact impairs the accuracy of measuring the pressure differential, hence the gas flow rate, especially at average and high (trunk) rates. The aforementioned phenomena prohibit the formation, in the method of pressure differential creation underlying the device operation, of a gas or liquid flow having uniform composition and other parameters and suitable for taking a continuous representative sample. Furthermore, to determine composition of fluid medium, the pipeline must be provided with additional equipment that is not associated with the flow metering equipment.

The object of the present invention is to reduce instantaneous error in the pressure differential formation, to improve the operating accuracy and stability of measurements, as well as to ensure formation of a gas or liquid flow section having homogeneous composition and suitable for taking a representative sample.

The object of the invention can be accomplished in a method for flow metering and forming a sample of a fluid medium in a pipeline, comprising passing a flow of the fluid medium through a grid of a straightening vane, a nozzle and a diffuser. On a path between the straightening vane and the nozzle a portion of the fluid medium enters a first static high pressure measuring cavity, on a section downstream of the nozzle a portion of the fluid medium enters a second static low-pressure measuring cavity, and in front of the diffuser a portion of fluid medium enters a side cavity for forming a sample of the fluid medium for subsequently determining the composition of said sample.

A device for implementing the inventive method comprises arranged successively along a common axis: a straightening vane, a cylindrical high-pressure chamber, a nozzle, a low-pressure chamber, a sampling chamber and a diffuser, wherein the high-pressure chamber communicates with an enclosing annular high-pressure measuring chamber, the low-pressure chamber communicates with an enclosing annular low-pressure measuring chamber, and the sampling chamber communicates with an enclosing annular chamber for additionally stirring the samples.

Cross section of the straightening vane comprises a grid having cells of a regular shape. For liquids, such as oil, the grids with square cells are generally provided. For gases, the straightening vane with a grid having hexagonal cells is used. However, grids having cells of another configuration are also possible. In all cases, thickness of cell walls should be minimal to avoid the effect of section shadowing that could give rise to velocity head loss and additional errors in flow metering and sample formation.

The high pressure chamber communicates with the enclosing annular high pressure measuring chamber, and the low pressure chamber communicates with the enclosing annular low pressure measuring chamber through longitudinal slot perforations having outwardly expanding cross sections.

In the annular chambers low and high pressure sensors are provided, as well as washing sleeves arranged preferably perpendicular to the common axis of the device.

The nozzle can be encompassed, on the external surface, by an annular chamber communicating with the low pressure measuring chamber. This embodiment can be used when the nozzle has thin walls and runs the danger of being deformed as the result of the pressure difference between the pipeline and the environment.

The low pressure chamber and the sampling chamber preferably have conical configuration with the slope ratio between the cone generatrix and the cone axis of 1/50-1/100.

Input end of the straightening vane and output end of the diffuser are coupled to the main pipeline and have the same diameter. The diffuser generally has a taper of 5-6° to enable recovery of pressure in the metering device without flow separation.

Design and operation of a device for implementing the inventive method are illustrated in the drawings, wherein Fig.1 shows a sectional view of a device for metering a flow rate and forming a sample of a fluid medium, connected to a pipeline. Fig.2 shows a cross sectional view of a straightening vane having square cells. Fig.3 shows a cross sectional view of a straightening vane having hexagonal cells.

A device for flow metering and forming a fluid medium sample is installed (e.g. using flange coupling 1) in a trunk pipeline (not shown) and comprises the following successively arranged components along a common axis: a straightening vane 2, a cylindrical high-pressure chamber 3, a nozzle 4, a low-pressure chamber 5, a sampling chamber 6 and a diffuser 7. The high-pressure chamber 3 is enclosed in and communicates with an annular high-pressure measuring chamber 8, the low-pressure chamber 5 is enclosed in and communicates with an annular low-pressure measuring chamber 9, and the sampling chamber 6 is enclosed in and communicates with an annular chamber 10 for additionally stirring the samples.

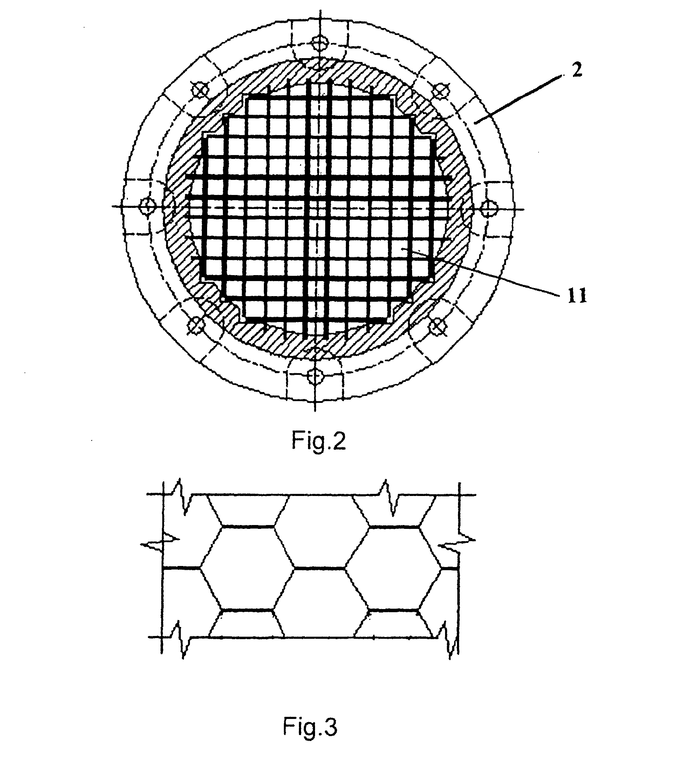

Cross section of the straightening vane 2 comprises a grid 11 having square cells (Fig.2) or hexagonal cells (Fig.3).

High pressure chamber 3 communicates with enclosing annular high pressure measuring chamber 8, and low pressure chamber 5 communicates with enclosing annular low pressure measuring chamber 9 through longitudinal slot perforations having outwardly expanding cross sections. The perforations are denoted by numerals 12 and 13, respectively.

Nozzle 4 is enclosed in an annular chamber 14 which communicates with the low pressure measuring chamber 9.

Sampling chamber 6 communicates, through slot perforations 15, with the enclosing annular chamber 10 for additionally stirring the samples.

Low pressure chamber 5 and sampling chamber 6 preferably have conical configuration with the slope ratio between the cone generatrix and the cone axis of 1/50-1/100. Diffuser 7 generally has taper of 5-6°.

Each of the annular chambers 8, 9, 10 and 14 comprises washing sleeves 16, the number of sleeves being dictated by operation conditions, density and adhesive power of the liquid passed through.

A device according to the invention operates as follows.

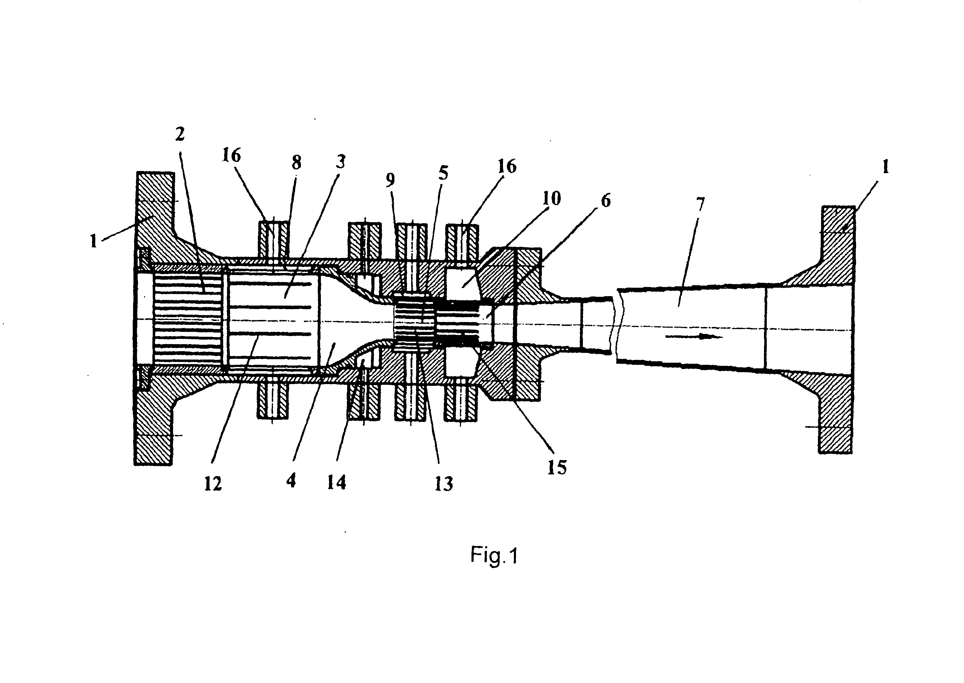

Initial turbulent flow of liquid or gas from a pipeline (general direction of the flow is shown by arrow in Fig.1) is divided by a grid 11 of a straightening vane 2 into a plurality of fine vortex jets whose turbulence has been already reduced by splitting the general flow vortex, as well as by friction and mass transfer at boundaries of micro-jets that cause loss of turbulence energy and mutual penetration of particles of adjacent micro-jet flows. The flow with substantially reduced turbulence enters a cylindrical high pressure chamber 3, and static pressure of the flow is transferred, through perforations 12 formed as longitudinal slots with expanding cross section (which dampen transverse pressure pulsations in the flow), into a coaxial annular chamber 8, where a high pressure sensor (not shown) is provided and where reliable value of the static pressure at the device inlet is determined. The reliability is provided by the number, cross-section shape and length of perforations 12, and by the distance between cylindrical walls of the chamber 3. The flow then enters a nozzle 4 where the macro-turbulence energy is further dampened by intensification of the mass transfer process between the flow micro-jets owing to the nozzle shape that increases loss of macro-turbulence energy of compressed micro-jets and averages the composition and other parameters of the micro-jets. The flow then enters a low pressure chamber 5, and the flow static pressure is transferred to a coaxial annual chamber 9 in which a low pressure sensor (not shown) is provided. The low pressure chamber 5 is configured as a right, circular, truncated cone with the slope ratio between the cone generatrix and the cone axis of 1/50-1/10; the smaller base of the cone being the inlet section of the chamber 5, and the greater base of the cone being located at the joint section between the low pressure chamber 5 and the sampling chamber 6.

The design of internal cavity of the low-pressure chamber 5 allows the device flow efficiency to be increased to a considerable extent depending on the density of the transported gas or viscosity of liquid, which considerably increases the flow rate measurement range, reduces macro-turbulence and thereby reduces the measured flow distortions at the measurement section where a reliable low static pressure value is transmitted, through perforations 13 configured as longitudinal, outwardly expanding slots, into a coaxial annular chamber 9 with a low pressure sensor provided therein.

The flow then enters a sampling chamber 6 which is enclosed in and communicates with an annular chamber 10 for additionally stirring the samples, and penetrates through slot perforations 15 into the stirring chamber 10, where owing to the gas or liquid transverse velocity component appeared at output of the slot perforations 15 the jets entered the chamber 10 from different slot perforations 15 are additionally mixed. Slot perforations 15 can be arranged in parallel with or at angle of up to 8° to the longitudinal axis of the device. The angled arrangement promotes a more uniform mixing of high viscosity fluids. Under the pressure in the pipeline, the mixed flow passes into a transport pipeline to deliver the sample to fluid composition analyzers (nor shown).

Samples taken for subsequent analysis particularly from the chamber 6 provide more reliable analysis results because constituents of the fluid in chamber 6 are most concentrated and uniformly distributed over the flow cross section.

The main flow of gas or liquid then enters a diffuser 7; the angle of the cone generatrix of chambers 5 and 6 is chosen such that no stalling turbulence disturbances appear when the flow passes though the diffuser 7 and enters the trunk pipeline, because the stalling turbulence disturbances could have a reverse effect on the flow in chambers 5 and 6 due to acoustic disturbance waves passing opposite to the moving flow, which could impair the measurement accuracy. This measure also allows determining an optimal slope of the generatrix of diffuser 7, hence its optimal size, based on the fact that kinetic and dynamic parameters of the flow entering the diffuser will be constant irrespective of the density of the transported gas or fluid, as provided by the choice of taper of chambers 5 and 6. Optimal taper of the diffuser is 5-6°. With a greater angle the flow swirling and separation could occur and impair the measurement accuracy, while with a smaller angle the diffuser length could be unreasonably long and hydraulic loss could increase drastically, this also impairing the measurement accuracy. The initial pressure of gas or liquid less the hydraulic loss is restored along the entire length of diffuser 7. Hydraulic losses in the device are:
for natural gas: no more than 0.04 kg/cm² at working pressure 75 kg/cm² and gas velocity 20 m/s at the device inlet;
for commercial oil: no more than 0.23 kg/cm² at working pressure 75 kg/cm², density 950 kg/m³ and oil velocity 3 m/s at the device inlet.

In an embodiment where nozzle 4 has a strictly defined shape and quite thin walls, the nozzle can be deformed in operation due to high pressure difference between the pressure inside the pipeline and the atmosphere. To prevent the deformation, the external surface of nozzle 4 is enclosed in annular chamber 14 which communicates with low pressure measuring chamber 9. In this case, substantially the same pressure will act upon the external and internal surfaces of nozzle 4 at any flow rate, and final formation of the flow before entering the measuring section will occur unaffected by the deformation component induced by the pressure difference.

Flow of a fluid medium generally carries a portion of solid particles which can adhere to internal walls of the annular chambers and change geometrical and rheological properties of the flow. A certain amount of adhesion can impair the accuracy of measurements; therefore the chambers are to be washed off from adhered particles through sleeves 16.

The inventive device makes it possible to reduce by an order of magnitude the measurement errors caused by inaccurate formation of pressure differential, to expand by 5 to 10 times the flow measurement range for one size, and to reduce the structure weight by about 20% as compared to conventional sampling and flow metering systems. The most substantial advantage provided by the invention is the reduction in hydraulic loss of the working pressure by 30-40 times for gas and 10-15 times for oil. This enables critical situations to avoided at trunk metering stations in situations where due to working pressure loss the flow cannot overcome 2 meters over-height when oil is delivered to a tank. The loss parameter can be easily computed and converted into direct gas and oil transport costs at known price of energy.

## Claims

1. A method for flow metering and forming a sample of a fluid medium in a pipeline, including passing a flow of the fluid medium through a grid of a straightening vane, a nozzle and a diffuser, wherein on a path between the straightening vane and the nozzle a portion of the fluid medium enters a first static high pressure measuring cavity, on a section downstream of the nozzle a portion of the fluid medium enters a second static low-pressure measuring cavity, and prior to the diffuser a portion of the fluid medium enters a side cavity for forming a sample of the fluid medium for subsequently determining the composition of said sample.

2. A device for flow metering and forming a sample of a fluid medium, comprising arranged successively along a common axis: a straightening vane, a cylindrical high-pressure chamber, a nozzle, a low-pressure chamber, a sampling chamber and a diffuser, said high-pressure chamber communicating with an enclosing annular high-pressure measuring chamber, the low-pressure chamber communicating with an enclosing annular low-pressure measuring chamber, and the sampling chamber communicating with an enclosing annular chamber for additionally stirring the samples.

3. The device according to claim 2, wherein the cross section of the straightening vane comprises a grid with square cells.

4. The device according to claim 2, wherein the cross section of the straightening vane comprises a grid with hexagonal cells.

5. The device according to claim 2, wherein said high-pressure chamber communicates with the enclosing high-pressure measuring chamber and said low pressure chamber communicates with the enclosing low pressure measuring chamber through longitudinal slot perforations having outwardly expanding cross section.

6. The device according to claim 2, wherein the nozzle is enclosed in an annular chamber communicating with the low-pressure measuring chamber.

7. The device according to claim 2, wherein the low-pressure chamber and the sampling chamber have conical configuration with the slope ratio between the cone generatrix and the cone axis of 1/50-1/100.

8. The device according to claim 2, wherein the diffuser has a taper of 5-6°.
